Europäisches Patentamt

European Patent Office

Office européen des brevets

(19)

(11) Veröffentlichungsnummer: **0 132 694**
**B2**

(12) **NEUE EUROPÄISCHE PATENTSCHRIFT**

(45) Veröffentlichungstag der neuen Patentschrift:
**31.10.90**

(51) Int. Cl.⁵: **A 61 M 25/00**, A 61 M 23/00

(21) Anmeldenummer: **84108063.3**

(22) Anmeldetag: **10.07.84**

(54) **Set bestehend aus schlauchförmigem Katheter oder Körpersonde und Mandrin.**

(30) Priorität: **02.08.83 DE 3327779**

(43) Veröffentlichungstag der Anmeldung:
**13.02.85 Patentblatt 85/07**

(45) Bekanntmachung des Hinweises auf die
Patenterteilung:
**29.10.86 Patenblatt 86/44**

(45) Bekanntmachung des Hinweises auf die
Entscheidung über den Einspruch:
**31.10.90 Patentblatt 90/44**

(84) Benannte Vertragsstaaten:
**AT BE CH DE FR GB IT LI LU NL SE**

(56) Entgegenhaltungen:
**WO-A-83/00631
DE-B-1 274 275
US-A-3 102 715
US-A-3 612 058
US-A-3 973 556**

**ELECTRONICS, Band 39, Nr. 5, 7. März 1966,
Seiten 100-101; "Coaxial connector called the
smallest"**

(73) Patentinhaber: **B. Braun-SSC AG
Gerliswilstrasse 74
CH-6020 Emmenbrücke (CH)**

(72) Erfinder: **Haacke, Claus, Dr.
Altstadt 6
D-3508 Melsungen (DE)**

(74) Vertreter: **von Kreisler, Alek, Dipl.-Chem. et al
Patentanwälte Von Kreisler-Selting-Werner,
Deichmannhaus am Hauptbahnhof
D-5000 Köln 1 (DE)**

EP 0 132 694 B2

**Beschreibung**

Die Erfindung betrifft einen Set der im Oberbegriff des Patentanspruchs 1 angegebenen Art.

Katheter und Körpersonden, die in menschliche Körperhöhlen eingeführt werden, müssen weich gestaltet sein, damit sie keine Verletzungen hervorrufen. Um einen Katheter oder eine Körpersonde gezielt in den Körper einzuführen, z.B. in eine Blutbahn, die Blase, den Magen oder den Darm, muss ein Versteifungselement in das Innere des Schlauches eingeführt werden, damit der Schlauch während des Einführens hinreichend steif ist. Ein solches Versteifungselement wird als Mandrin bezeichnet.

Es ist vielfach üblich, zuerst das Versteifungselement in den Körper einzuführen und dann den Schlauch darüberzuschieben. Danach wird der dünne Mandrin, der auch als Seldinger-Draht bezeichnet wird, wieder aus dem Schlauch herausgezogen.

Bei einem bekannten Set der eingangs genannten Art (Zeitschrift Anaesthesist, 29, 498 bis 503) besteht der Mandrin aus einer Drahtseele, die von einem schraubenförmig oder wendelförmig gewickelten einzigen Aussendraht umgeben ist. Die Drahtseele bildet einen Sicherheitsdraht und bestimmt massgeblich die Steifigkeit des Mandrins, während der Aussendraht wesentlich dünner ist und eine grosse Flexibilität hat. Die Windungen des Aussendrahtes liegen dich aneinander an, so dass die Steigung des Aussendrahtes 1:1 beträgt, d.h. dass der Abstand, den die Mittelachsen benachbarter Windungen voneinander haben, gleich der Stärke des Aussendrahtes ist. Der Aussendraht verläuft somit fast quer zur Längsrichtung des Mandrins. Wird der Mandrin in einen engen Schlauch eingeschoben, dann ergibt sich eine relativ grosse Reibung zwischen den zahlreichen Windungen des Aussendrahtes und der Innenwand des Schlauches.

Es ist ferner bekannt, einen Führungsdraht entweder als Einzeldraht oder als Litze in einen dickwandigen Kunststoffmantel einzubetten (EP-PS 0 014 424, DE-GM 8 123 912, DE-GM 8 132 839). Der Kunststoffmantel hat eine zylindrische Aussenfläche, mit der er sich vollflächig an die Innenwand eines dünnen Schlauches anlegen kann. Hierdurch entsteht ein grossflächiger Flächenkontakt zwischen Mandrin und Schlauch, so dass es oft schwierig ist, Mandrin und Schlauch relativ zueinander zu verschieben, weil die Reibung zu gross wird. Selbst bei Verwendung besonders gleitfähiger Materialien, wie Fluorethylenpropylen-Copolymer (FEP) oder Polytetrafluorethylen (PTFE), kann die Haftung der beiden Teile wegen der Oberflächenadhäsion der beiden glatten Polymerflächen und infolge der Kurven, die der Schlauch im menschlichen Körper annimmt, nur schwer überwunden werden. Es ist daher sehr schwierig, den Mandrin aus einem engen Schlauch herauszuziehen.

Die schlechte Verschiebbarkeit des Mandrins im Schlauch führt auch zu einer erhöhten Montagezeit im Herstellerbetrieb. In der Regel werden die Schläuche mit eingeschobenem Mandrin geliefert.

Der Erfindung liegt die Aufgabe zugrunde, einen Set der eingangs genannten Art zu schaffen, bei dem der Mandrin sich in dem engen Katheter oder der Körpersonde leichter verschieben lässt.

Die Lösung dieser Aufgabe erfolgt erfindungsgemäß mit den Merkmalen des Patentanspruchs 1.

Bei dem Mandrin des erfindungsgemäßen Sets sind mehrere Aussendrähte um die Drahtseele herum nach Art einer Verseilung gewickelt. Die Aussendrähte bedecken den gesamten Umfang der Drahtseele, haben aber eine relativ grosse Steigung. Unter Steigung wird das Verhältnis der Höhe einer Windung zur Stärke des Aussendrahtes verstanden. Infolge der grossen Steigung verlaufen die Aussendrähte mit einer erheblichen Komponente in Längsrichtung des Mandrins, also in Schieberichtung. Am Umfang des Mandrins treten die Konturen der Aussendrähte plastisch hervor. Dies bedeutet, dass der Mandrin keine zylindrische Oberfläche hat. Die Oberfläche des Mandrins wird vielmehr von den Aussenseiten der einzelnen Aussendrähte gebildet. Zwischen jeweils zwei Aussendrähten verläuft eine schraubenförmige Furche. Infolge dieser Ausbildung der Oberfläche des Mandrins wird die Oberflächenadhäsion mit dem Schlauch, in den der Mandrin eingeschoben wird, erheblich herabgesetzt. Da die Aussendrähte eine grosse Steigung haben und mit starker Komponente in Längsrichtung ausgerichtet sind, entstehen geringe Reibungs- und Adhäsionswiderstände zwischen Mandrin und Schlauch.

Ein wesentlicher Vorteil der Erfindung besteht darin, dass die Anzahl und der Durchmesser der Aussendrähte sowie der Durchmesser der Drahtseele in zahlreichen Variationen aufeinander abgestimmt werden können, um einen Mandrin mit einer für den jeweiligen Anwendungsfall günstigen Steifigkeit zu erhalten. Durch geeignete Wahl der Drahtseele und der Aussendrähte kann man den Gesamtdurchmesser des Mandrins und auch dessen seitliche Flexibilität sehr gut regeln.

Die Anzahl der Aussendrähte beträgt vorzugsweise fünf bis acht. Je grösser die Anzahl der Aussendrähte ist, um so stärker kann die Drahtseele gemacht werden. Die Stärke der Drahtseele bestimmt die Steifigkeit des Mandrins. Wenn man einen Mandrin mit geringerer Steifigkeit erhalten will, wählt man eine dünnere Drahtseele und eine kleinere Anzahl von Aussendrähten.

Die Steigung der Aussendrähte beträgt vorzugsweise mindestens 7:1, sie kann aber wesentlich grösser sein, z.B. 12:1 oder noch höher.

Um ein Eindringen von Körperflüssigkeit in das Innere des Mandrins zu vermeiden, ist gemäss einer vorteilhaften Weiterbildung der Erfindung vorgesehen, dass die Aussenseiten der Aussendrähte mit einem Kunststoff beschichtet sind, dessen Schicht so dünn ist, dass die Konturen der Aussendrähte auf der Oberfläche erhalten bleiben. Die Kunststoffschicht ist dünn und möglichst gleichmässig, so dass die Drahtstruktur auf der Oberfläche des Mandrins plastisch hervortritt. Die Furchen zwischen den Aussendrähten werden also nicht mit dem Kunststoff aufgefüllt, so dass die Aussenkontur des Drahtes sich von der zylindrischen Innenkontur des Schlauches (des Katheters oder der Körpersonde) unterscheidet.

Vorzugsweise besteht die die Enden der Außendrähte einschließende Plombe aus Kunststoff oder Metall. Die Plombe dient dazu, die Enden der Aussendrähte zusammmenzuhalten, sie kann durch Schweissen, Löten oder Umspritzen erzeugt werden.

Die Drahtseele braucht sich nicht über die gesamte Länge des Mandrins zu erstrecken. Sie kann vielmehr vor dem patientenseitigen Ende des Mandrins enden, so dass dieser Endabschnitt gegenüber der übrigen Länge des Mandrins eine erhöhte Flexibilität hat. Der Endabschnitt kann auch J-förmig umgebogen bzw. vorgespannt sein.

Im folgenden werden unter Bezugnahme auf die Zeichnungen Ausführungsbeispiele der Erfindung näher erläutert.

Es zeigen:

Fig. 1 eine Seitenansicht des patientenseitigen Endes eines Mandrins mit einer Schnittdarstellung des Endes eines Katheters und

Fig. 2 bis 4 Querschnittsdarstellungen verschiedener Ausführungsbeispiele des Mandrins.

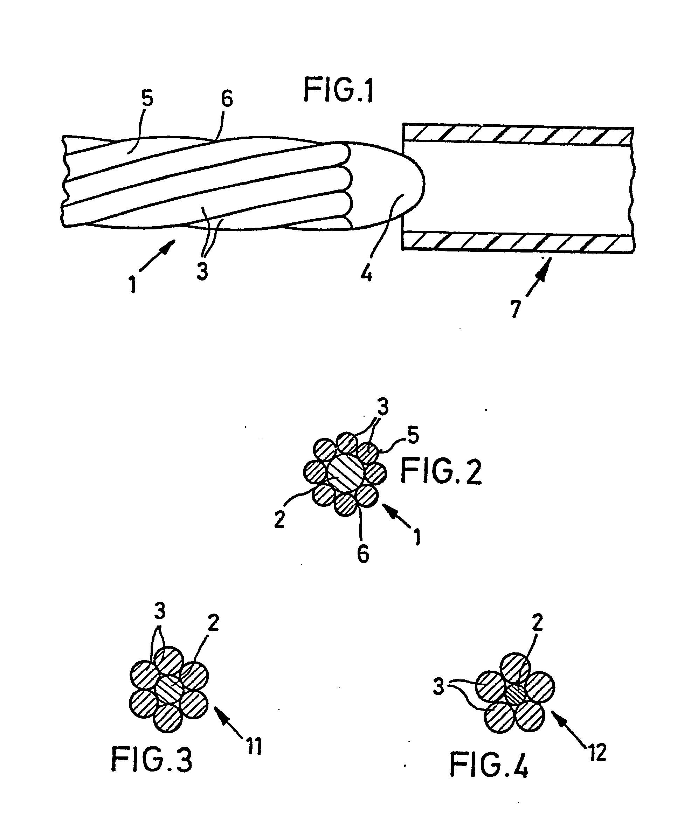

Der in Fig. 1 dargestellte Mandrin 1, dessen Querschnitt in Fig. 2 dargestellt ist, weist eine Drahtseele 2 aus einem langgestreckten geradlinigen Draht mit rundem Querschnitt auf. Um die Drahtseele 2 herum sind sieben Aussendrähte 3 angeordnet. Die Aussendrähte 3 sind schraubenförmig um die Drahtseele 2 herumgewickelt, wobei die Steigung eines jeden Aussendrahtes 3 etwa 12:1 beträgt. Die Aussendrähte 3 haben untereinander gleichen Durchmesser. Diese Durchmesser sind kleiner als der Durchmesser der Drahtseele 2. An dem patientenseitigen Ende des Mandrins 1 sind die Aussendrähte 3 in einer Plombe 4 zusammengefasst und miteinander verschweisst oder verlötet. Die Plombe 4 hat an ihrem Ende die Form einer abgerundeten Kuppe.

Der Mandrin 1 ist an seiner Umfangsfläche, an der sich die etwa halbkreisförmigen Aussenseiten der Aussendrähte 3 abzeichnen, mit einer Kunststoffbeschichtung 5 von im wesentlichen gleichmässiger Stärke versehen. Wie aus Fig. 3 ersichtlich ist, folgt die Aussenkontur der Beschichtung 5 der Konturr der Aussendrähte 3, so dass sich zwischen zwei Aussendrähten 3 jeweils eine wendelförmige Furche 6 befindet.

Der Mandrin 1 wird gemäss Fig. 1 in den schlauchförmigen Katheter 7 eingeschoben. Das Katheterlumen (der Innendurchmesser) entspricht im wesentlichen dem Aussendurchmesser des Mandrins 1. Da sich der Mandrin 1 nur mit den Wölbungen der Beschichtung 5 an der Innenwand des Katheters 7 abstützt, gleitet er ohne grösseren Haftwiderstand in dem Katheter.

Bei dem Ausführungsbeispiel der Fig. 2, bei welchem die Drahtseele 2 einen grösseren Querschnitt hat als die Aussendrähte 3, ist der Mandrin 1 relativ steif. Wenn der Mandrin eine grössere Flexibilität haben soll, wählt man das Ausführungsbeispiel gemäss Fig. 3, bei welchem die Drahtseele 2 den gleichen Durchmesser hat wie die Aussendrähte 3. Bei dem Mandrin 11 in Fig. 3 sind nicht sieben Aussendrähte 3 gleichmässig um den Umfang des Drahtkernes 2 angeordnet, sondern nur sechs Aussendrähte.

Eine noch grössere Flexibilität hat der Mandrin 12 in Fig.4. Bei diesem Mandrin sind fünf Aussendrähte 3 gleichmässig um die Drahtseele 2 herum angeordnet. Der Durchmesser der Drahtseele 2 ist kleiner als der Durchmesser der Aussendrähte 3.

Bei allen Ausführungsbeispielen stehen die Aussendrähte 3 in direktem Berührungskontakt mit der Drahtseele 2. Die Aussendrähte sind nach Art einer Verseilung wendelförmig um die Drahtseele 2 herumgewickelt. Ein besonderer Vorteil des Mandrins besteht in der einfachen Herstellungsmöglichkeit in bekannter Verseiltechnik.

**Patentansprüche**

1. Set bestehend aus einem schlauchförmigen Katheter oder einer Körpersonde und einem daran angepaßten Mandrin (1), der seinerseits aus einer mit Außendraht schraubenförmig umwickelten Drahtseele (2) besteht, wobei das Ende des Außendrahts (3) in einer Plombe (4) eingeschlossen ist, dadurch gekennzeichnet, daß bei dem Mandrin mindestens vier Außendrähte (3) vorgesehen sind, die mit hoher Steigung von mindestens 5:1 um die Drahtseele (2) gewickelt sind.

2. Set nach Anspruch 1, dadurch gekennzeichnet, dass die Anzahl der Aussendrähte fünf bis acht beträgt.

3. Set nach Anspruch 1 oder 2, dadurch gekennzeichnet, dass die Steigung der Aussendrähte (3) mindestens 7:1 beträgt.

4. Set nach einem der Ansprüche 1 bis 3, dadurch gekennzeichnet, dass die Aussenseiten der Aussendrähte (3) mit einer Kunststoffbeschichtung (5) beschichtet sind, die so dünn ist, dass die Konturen der Aussendrähte (3) auf der Oberfläche erhalten bleiben.

5. Set nach einem der Ansprüche 1 bis 4, dadurch gekennzeichnet, dass die Plombe (4) aus Kunststoff oder Metall besteht.

**Revendications**

1. Ensemble constitué par un cathéter en forme de tuyau ou une sonde corporelle et par un mandrin (1), qui est adapté au cathéter ou à la sonde et est constitué, pour sa part, par une âme (2) formée d'un fil, entouré d'un fil extérieur posé selon un enroulement hélicoïdal, l'extrémité du fil extérieur (3) étant insérée dans une masse de scellement (4), caractérisé en ce qu'il est prévu dans le mandrin au moins quatre fils extérieurs (3), qui sont enroulés avec un pas important égal au moins à 5:1, autour de l'âme (2) formée d'un fil.

2. Ensemble selon la revendication 1, caractérisé en ce que le nombre des fils extérieurs est de cinq à huit.

3. Ensemble selon la revendication 1 ou 2, caractérisé en ce que le pas des fils extérieurs (3) est égal à au moins 7:1.

4. Ensemble selon l'une des revendications 1 à 3, caractérisé en ce que les faces extérieures des fils extérieurs (3) sont recouvertes par une gaine en matière plastique (5) qui est suffisamment mince pour que les contours des fils extérieurs (3) restent conservés au niveau de leur face.

5. Ensemble selon l'une des revendications 1 à 4, caractérisé en ce que la masse de scellement (4) est constituée par une matière plastique ou un métal.

**Claims**

1. A set consisting of a hose-shaped catheter or a body probe and a mandrel (1) adapted thereto which in turn consists of a wire core (2) helically wrapped with external wire, the end of said external wire (3) being enclosed in a seal (4), characterized in that said mandrel is provided with at least four external wires (3) wrapped about said wire core (2) at a high pitch of at least 5:1.

2. The set according to claim 1, characterized in that the number of external wires is at least five to eight.

3. The set according to claim 1 or 2, characterized in that the pitch of said external wires (3) is at least 7:1.

4. The set according to one of claims 1 to 3, characterized in that the outsides of the external wires (3) are coated with a plastic layer (5) which is thin so that the contours of the external wires (3) are maintained on the surface.

5. The set according to one of claims 1 to 4, characterized in that the seal (4) consists of plastics or metal.

FIG.1

FIG.2

FIG.3

FIG.4